# EUROPEAN PATENT SPECIFICATION

(11) **EP 1 439 370 B1**
(45) Date of publication and mention of the grant of the patent: **25.07.2007**
(21) Application number: 04009865.9
(22) Date of filing: 17.04.2000
(51) Int. Cl.: G01B 3/10, G01B 21/02

(54) **Electronic measuring tape**
Elektronisches Messband
Ruban de mesure electronique

(30) Priority: 16.04.1999 GB 9908784
(43) Date of publication of application: 21.07.2004
(62) Divisional of application: 00927423.4
(73) Proprietor: THE SECRETARY OF STATE FOR TRADE AND INDUSTRY, London SW1 0ET (GB)
(72) Inventor: Turner, Nicholas Paul, Oxfordshire, OX12 8 QJ (GB)
(74) Representative: Jehan, Robert

(56) References cited:
- EP-A- 0 103 216
- GB-A- 2 023 290
- US-A- 2 966 797
- US-A- 4 575 944

## Description

The present invention relates to an electronic measuring tape, for example a flexible tailor's tape measure. This is a divisional of European patent application no. 00927423.4.

A conventional fabric tape measure requires user judgement in accurate reading and user reliability in recording correctly the measurements which have been taken. The readings can also be subject to user bias, unintentional or otherwise, and mistakes can be made in recording a large number of measurements.

Electronic measuring tapes are known. For example, EP-A-0740126 discloses a digital tape measure with regularly-spaced markers which are detected by electronic sensing means. In addition, US-A-5,743,021 discloses a digital length gauge with a tape having a series of uniformly spaced holes which drive pins on a sprocket connected to an optical encoder. The need to provide markers and/or holes in a tape imposes limitations on the weight, thickness, strength and flexibility of the material used for the tape.

GB-A-2023290 discloses a tape measure with a tape housing incorporating an electronic calculator, a tape exit opening, and an internal take-up spindle for the tape which is driven by a motor and provided with an optical device co-operating with a series of holes in a disk rotated by movement of the tape for sensing the length by which the tape has been extended or further extended.

US-A-4575944 discloses an electronic digital tape rule consisting of a housing, a tape reel having an extendible and flexible measuring tape, a battery case, an actuator, a detector, a counter, a display and a brake. The actuator comprises a friction roll rotatably mounted on a shaft fixed at an inner wall of the housing, a disc disposed on the upper end of the roll and rotatable by the friction of the roll, a plate fixed on the upper end of the roll and having a hole with a pair of contact plates attached on the two side walls of the hole, a spring mounted between the disc and the plate for constantly pressing the disc against the roll and an actuating rod disposed upright on the upper side of the disc and having its upper end engaging with the hole.

EP-A-0103216 discloses a portable measuring device for measuring wood by hand comprises callipers for the diameter measurement, on the displaceable measuring leg of which a length measuring device with a pull-out measuring tape is arranged. The callipers and the length measuring device are each assigned an automatically operating pick-up device for the production of an electric diameter or length signal. The distance pick-up device comprises a Wiegand strip inside the guide tube of the callipers and an associated Wiegand sensor on the movable measuring leg of the callipers. The length pick-up device comprises a measuring wheel which is surrounded by the steel measuring tape and on which the measuring tape, as a result of an axial magnetic polarisation of the measuring wheel on its circumference, adheres free of slip. The rotary movement of the measuring wheel corresponding to the pull-out movement of the measuring tape is detected by a shaft-angle encoder which comprises permanent magnets on the measuring wheel and associated magnetic field sensors on the movable measuring leg.

US-A-4999924 discloses a tape measurement marking device attached to a measuring tape or built into a conventional tape housing. The device includes a sighting plane for alignment with the desired measurement mark on the tape; a structure for receiving and directing a hammer blow to a marking blade; and a marking blade that produces a dimensionless mark precisely at the desired measurement, a void to place the tape hook in, the void having an edge to hook the tape onto, the edge coinciding with the desired measurement mark; when the device is used on an existing tape, mechanisms for assembling, sliding, and clamping it on the tape are included.

US-A-2966797 discloses another version of electronic measuring tape.

The present invention seeks to overcome or reduce one or more of the disadvantages of the prior art.

According to a first aspect of the present invention, there is provided an electronic tape measure device as specified in claim 1.

An advantage of this arrangement is that a plain tape can be used. Moreover, a fabric tape of the type used by tailors or in dressmaking may be used.

Over a period of time it is possible for the tape to stretch and/or for tape slippage to develop and/or for the measuring accuracy of the device to drift. In order to calibrate prior art electronic devices, it would be necessary to measure known distances with the devices, which could be a long and complicated procedure.

The present invention also seeks to provide a quick and simple calibration method.

The preferred device is a hand-held measuring tape on a spring loaded reel, similar in mechanical operation to a carpenter's steel tape measure but made of a soft, pliable and substantially non-stretchable material which can be safely applied to human skin and which remains flexible after repeated bending.

The amount of tape pulled out from the device is detected electronically by an encoder and the measurements made by the device are fed directly into a computer when triggered by a push button.

The output from the encoder may be treated in the same way as the output from the encoders in a conventional PC mouse and is fed by flexible cable to a standard COM port on a PC in the same way that a mouse is connected to a PC. A further option is to fit telemetry electronics to the case and the PC in order to obviate the need for a cable.

The device is supplied with software modules to interpret the signals arriving at the COM port according to user need. One or more push buttons on the device may signal the beginning and end of a measurement.

A thumb operated push button may be located on the device and computer software provided which interprets one push of the button as a zero reading with the tape retracted and the next push as a measurement. The electronic pulses from the device, detected by the PC, are converted to length measurements by a calibration factor stored in the computer program.

It is also envisaged that communication with a computer could be wireless, for example by infra-red, ultrasound or radio frequency signals.

The calibration factor is determined by using the device to measure known distances; one such distance being part of the periphery of the device itself. Alternatively, calibration artefacts such as cylinders of known circumference could be supplied with the device. Data handling can be customised to user need through supply of appropriate software modules.

Preferred embodiments of the present invention will now be described, by way of example only, with reference to the accompanying drawings, of which:
Figure 1 is a perspective view of an electronic measuring tape device in accordance with a first embodiment of the present invention;
Figure 2 is a cross-sectional side view, or an enlarged scale, of the device of Figure 1;
Figure 3 is a sectional plan view of the device of Figure 1;
Figure 4 shows one method of use of the device of Figure 1;
Figure 5 is a schematic perspective view of an electronic measuring tape device in accordance with a second embodiment of the present invention;
Figure 6 is a view of the device of Figure 5 in self-calibration mode;
Figure 7 is a cross-sectional side view of a modified tape device; and
Figure 8 is a perspective view of the device of Figure 7 with the tape extended.

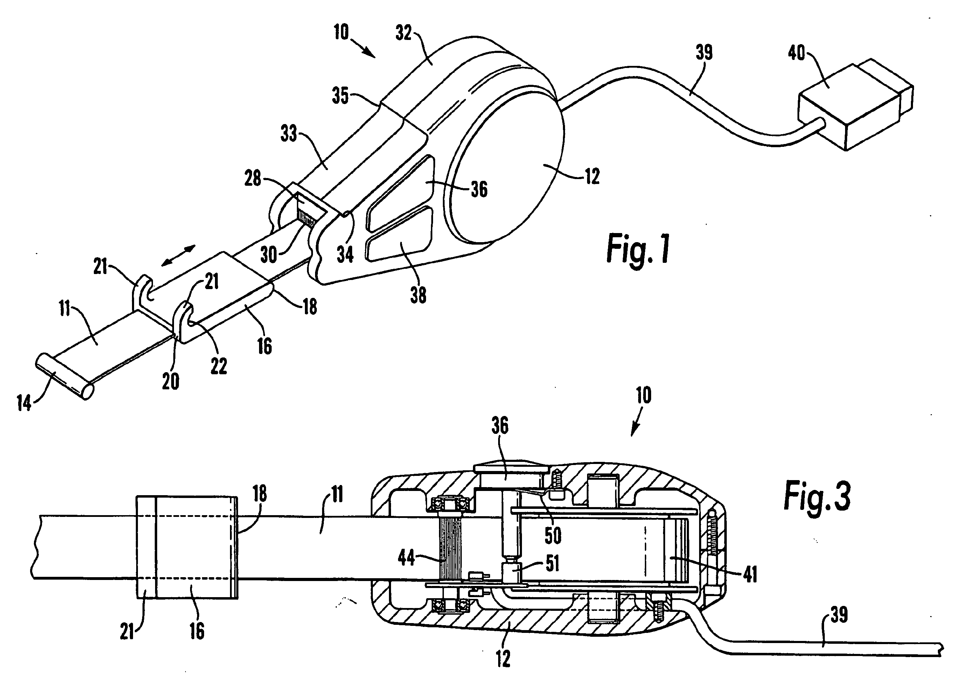

Referring now to the drawings, Figs. 1 to 4 show a measuring device 10 comprising a tape 11 which is of plain, unmarked, flexible fabric material and which can be withdrawn from and returned to a housing 12. Typical dimensions of housing 12 are: length 115 mm, width 78.8 mm and depth 52 mm. Tape 11 is typically 15-20 mm wide. The end of the tape 11 is provided with a fixed round bar member 14. A saddle or bracket member 16 is arranged to slide along the tape 11 with a frictional grip, so that, when moved by a user, it remains in the same position on the tape until it is adjusted again. Bracket member 16 comprises a rounded end 18 nearer the housing 12, and a hooked end 20 nearer to member 14, the hooked end housing two hook elements 21 each with an inner curve 22 matched to the curvature of the bar member 14. To receive the tape 11, the member 16 is provided with an interior through slot extending between ends 18 and 20.

Housing 12 comprises two interfitting halves which define a front surface 28 with a slot 30 for passage of the tape 11. The housing has a curved side edge 32 which extends substantially all the way around from the bottom of front surface 28 to the top of the front surface. Edge 32 is slightly wider than the tape 11 and has, at its end adjacent the top of front surface 28, a recess 33 with curved ends 34, 35. The curvature of front curved end 34 corresponds to the curvature of the bar member 14. The curvature of the rear curved end 35 corresponds to the curvature of the rounded end 18 of the saddle 16.

Housing 12 also has buttons 36, 38 for controlling operation of the device, and a five metre length of cable 39 with a plug 40 for connection to a power supply and communication with a computer (not shown).

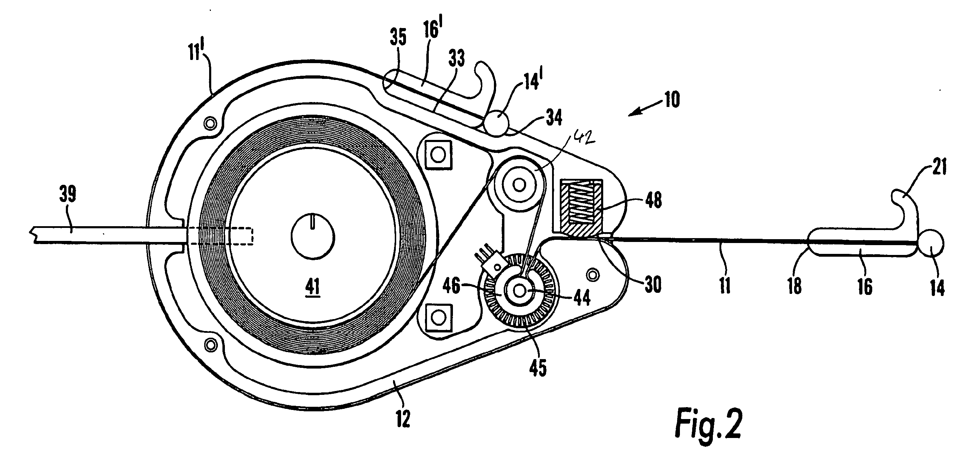

Inside the housing 12, the tape 11 is wound around a sprung storage drum 41, Figure 2. From here it passes around an idle roller or pulley 42 and then around a knurled shaft 44 which is fixedly attached to an encoder wheel 45. The wheel 45 is part of an encoder device, the output of which is connected to cable 39. In a preferred embodiment, the shaft 44 has a diameter of 6.366 mm so that one revolution of the shaft corresponds to 20 mm of linear movement of the tape 11. An optical encoder with apertures at spacing of 0.5 mm enables a resolution of better than 1mm to be obtained. In another embodiment a larger shaft with a diameter of up to 25 mm may be used.

The tape 11 then passes between the inner surface of a floating collar 46, in the form of an almost-closed ring, and the knurled surface of shaft 44; this serves to keep the tape in non-slipping contact with the shaft. It will be seen that the collar 46 constitutes a tape capture mechanism. The tape then passes past a spring biased element 48 forming the exit slot 30. Element 48 serves as a governor, ie as a device to restrict the speed at which the tape 11 passes. Element 48 also serves to reduce the amount of dirt entering the housing 12. In case any dirt does enter the housing 12, it may be taken apart and shaft 44 may be removable for cleaning purposes to prevent any deterioration in the performance of the encoder.

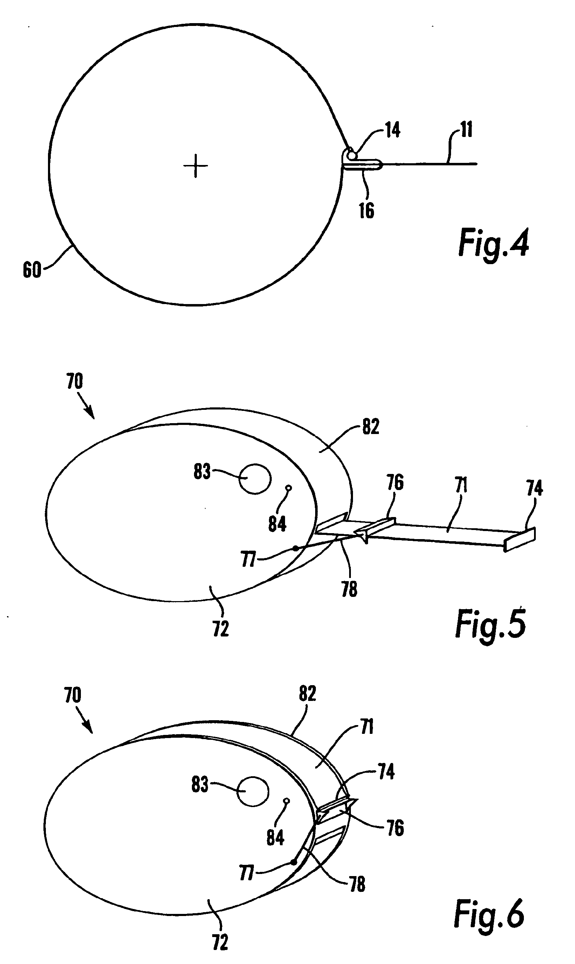

As shown in Figure 3, button 36 acts against a leaf spring 50 to actuate a corresponding micro switch 51. In a preferred arrangement, button 36 operates to reset the electronic measurement function of the device. Not visible in Figure 3 is button 38, which has its respective leaf spring and micro switch and preferably serves to actuate a "data send" operation of the device or to indicate a gap between different sets of data.

In use, the bar member 14 is successively placed at the two extremeties of a dimension to be measured, and the buttons 36, 38 are operated to supply the relevant encoder information to the computer. In cases where the device 10 itself can be maintained stationary at one of the extremeties (or in line with the measurement), this operation is straightforward. It can be of assistance to place the saddle member 16 against the end of an article or dimension to be measured which is nearer to the device 10, but the distance between the device 10 and the saddle member needs to be kept constant throughout the process. This could be conveniently arranged by keeping end 18 tight against slot 30.

For other measuring processes, the method illustrated in Figure 4 can be used. Here, the circumference of circle 60 represents a dimension to be measured, e.g. the waist measurement of a person. The datum level for this measurement is set by retracting the tape 11 into the device 10 as far as possible so that only members 14 and 16 are visible with the end 18 of the latter engaging slot 36. A length of the tape 11 is then withdrawn from the device and wrapped around the person's waist, bar member 14 is latched in the hook elements 21, and the tape is then suitably tightened around the waist by moving saddle member 16 along the tape. Upon completion the of the fitting, bar member is released and the tape can be gently fed back onto drum 41 until end 18 engages slot 30, care being taken to ensure that saddle member 16 does not move along the tape during this process. The output of the device now represents the required dimension. Note that it is not necessary to locate the housing 12 directly against the person's waist, which could be inconvenient. Alternatively, the datum level could be set at or after the end of the measurement procedure, by moving the end 20 of the saddle member 16 against the person being measured; of course the length of saddle member 16 and/or the length correction required when member 14 is placed in hook elements 21 may be automatically compensated for in the computer software.

From time to time, it may be necessary to undertake a calibration process. The device 10 provides a convenient facility for this to define a known distance, by wrapping the tape around the side edge 32 to the position 11' illustrated in Figure 2. Here, the saddle member shown as 16' and the bar member shown as 14' fit precisely in recess 33 between curved ends 34, 35. It may be detected how many encoder pulses correspond to a fixed distance, e.g. to detect whether any tape slippage is occurring.

The above-described device has several advantages. The use of a fully flexible fabric tape allows difficult measurements to be taken, e.g. of human beings or animals. Other awkward dimensions may also be measured. The employment of frictional engagement between the tape and the shaft 44 means that a plain unmarked tape can be used without holes or other openings. The use of both the end member 14 and the saddle member 16 to define the extremeties of a dimension to be measured improve both the accuracy and the convenience of the measurement process. Moreover, the device may be used with a standard personal computer. In addition the provision of dimension of known extent, ie the length of edge 32, provides an extremely convenient self-calibrating feature and avoids the need to provide separate measurement standards.

Various modifications can be made to the above-described arrangement.

For example, saddle member 16 can be omitted, in which case the front surface 28 of the housing 12 can be used to define one extremity of the dimension to be measured. Only part of the periphery of the housing 12, or the dimension of some other part may be used to calibrate the device; alternatively the calibration feature may be omitted completely. The device may comprise one or more lights and/or sound emitting elements (eg giving an electronic beep) to indicate satisfactory operation, in particular completion of a data transfer process. Further control members, eg an on-off switch may also be provided. As a background example and not as an embodiment of the invention, instead of a frictional collar 46, the tape 11 may be urged against the encoder shaft by a pinch wheel (not shown). Instead of a spring-biased member 48 being provided, the tape 11 may emerge for the housing between two relatively-small guide rollers. Any suitable rotary encoder means may be used in association with shaft 44.

Recess 13 may be dimensioned to receive bar member 14 alone.

The tape may be of other materials e.g. of metal.

Figures 5 and 6 show a device 70 in accordance with a second embodiment of the present invention. The device 70 has a rounded shape that is designed to fit comfortably in the palm of the hand. It has a flexible tape 71, intended to be similar in use and feel to a conventional tailor's fabric measuring tape. The tape is manufactured in a material with properties of softness, non-toxicity, strength, flexibility and minimal stretching. There need be no markings on the tape, though a conventional scale may be added. The tape has a generally flat rubber end stop 74. In this embodiment, a light but sturdy saddle 76 is hinged by arms 78 to the housing 72 at 77 and resets on the tape. A zero measurement is deemed to be when the end stop 74 rests at the end of the saddle 76, ie when the tape is retracted.

In use, the end of the saddle 76 marks one end of the distance to be measured and the end stop marks the other end. Circumferential measurements are made by drawing the tape from its zero position, around the object to be measured and touching the end stop on the end of the saddle again. The measured distances could be introduced, for example, into a spreadsheet for easy manipulation of the data.

In this embodiment, the housing 72 can be used as a calibration check by folding the saddle 76 upwards against the exterior periphery 82 of the housing and drawing the tape 71 around the housing until the end stop 74 touches the end of the saddle 76. The device 70 is supplied with a calibration value for this measurement. As with device 10, further calibration standards can be supplied with the device if required.

The device has a push button 83 and an indicator light 84. It is envisaged that a display may also be provided on the housing 72 and that there may also be a degree of data processing within the unit, possibly to the extend of enabling the unit to be totally self-contained in terms of distance measurement and display.

The features and modifications of the first and second embodiments may be interchanged as desired.

In a further modification, not shown, a saddle of similar shape to saddle 16 is fixedly attached to the housing of the device. In this case the free end of the saddle can be used to locate one extremity of a dimension to be measured. In a calibration measurement, member 14 may fit in the hook elements 21, as with the circumferential measurements of the first embodiment.

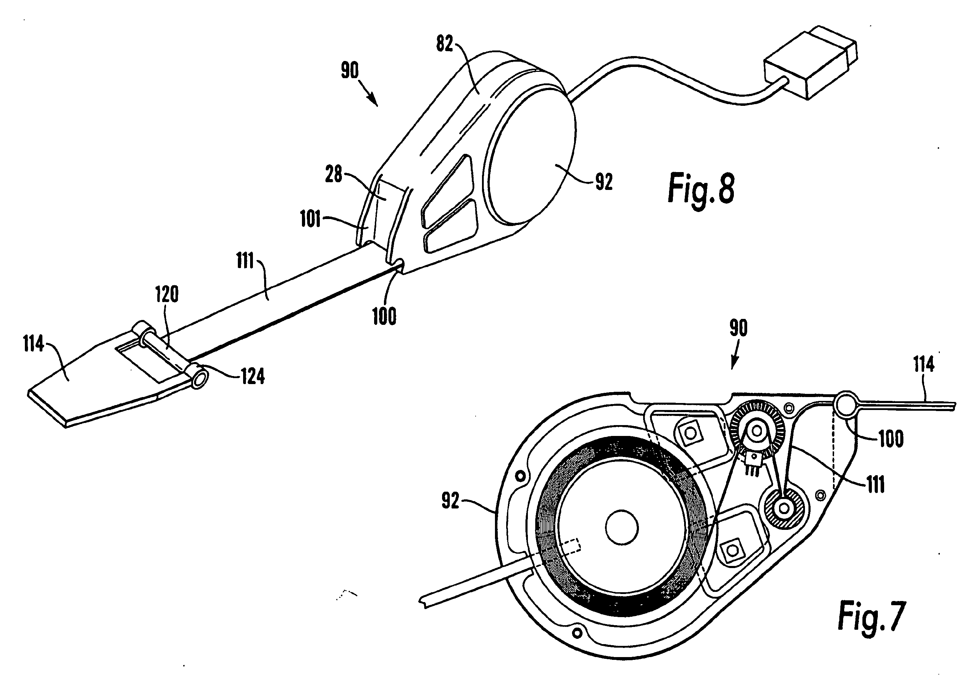

In yet another modification, Figures 7 and 8, to replace the hook elements 21, a device 90 has recesses 100 formed in projections 101 from the sides of the front surface 28 of a housing 92. Attached to the end of tape 111 is a shaped member 114. The end 120 of member 114 which is nearer to the housing 92 has a circular bar member 124 which matches the curvature of recesses 100. Thus member 124 fits in recesses 100:
(i) in the storage position shown in Figure 7;
(ii) in a calibration position in which a length of tape 111 is wrapped once around the entire periphery of housing 92; and
(iii) in a circumferential measurement position, e.g. when measuring a person's waist.

## Claims

1. An electronic tape measure device (10, 70, 90) comprising a tape (11, 71, 111) which passes around a rotatable shaft (44), the shaft being connected to an encoder device (45) for measuring the amount of tape withdrawn and wherein means (46) are provided for constraining the tape to engage the shaft in a frictional manner **characterised in that** the constraining means comprises a collar (46) arranged around the shaft (44) and the tape passes between the collar and the shaft.

2. A device according to claim 1, wherein the tape (11) has a fixed end member (14) and a relatively-movable member (16) which is movable by a user along the tape but remains in an adjusted position relative to the tape.

3. A device according to claim 2, wherein the relatively-moveable member (16) includes hook means (21), the end member (14) being arranged to fit within said hook means.

4. A device according to any preceding claim, wherein the device comprises a housing (12, 22) the exterior of which defines a path (32, 82) along which the tape may be placed to define a known dimension for calibration purposes.

5. A device according to claims 2 and 4 wherein the end of the path is defined by a recess (33) which is dimensioned to fit either the end member (14) or a combination of the end member (14) and the relatively-movable member (16) in contact with each other.

6. A device according to claim 1, wherein the device comprises a housing (72) and a member (76) is attached to the housing to define a measuring position.

7. A device according to claim 6, wherein the member (76) is pivotally attached to the housing (72).

8. A device according to claim 6 or 7, wherein the exterior of the housing (72) defines a path (82) along which the tape (71) may be placed to define a known dimension for calibration purposes.

9. A device according to claims 7 and 8, wherein said member (76) is pivotable to a first position for defining said measuring position and pivotable to a second position to define the end of said exterior calibration path (82).

10. A device according to claim 6, wherein said member is fixedly attached to the housing.

11. A device according to claim 10, wherein the said member includes hook means.

12. A device according to claim 10, wherein the member comprises one or more recesses arranged at or adjacent the portion (28) of the housing from which the tape (111) emerges.

13. A device according to any of claims 6-12, wherein the exterior of the housing has a formation (33, 76, 100) relative to which, after a length of tape has been withdrawn and wrapped around the periphery (32, 82) of the housing, the member may be accurately located in a reputable manner.

14. A method of measuring a distance using a device (10,70) according to any preceding claim.

## Patentansprüche

1. Elektronisches Bandmessgerät (10, 70, 90) mit einem Band (11, 71, 111), welches um eine drehbare Welle (44) herum läuft, wobei die Welle mit einer Kodiereinrichtung (45) zum Messen der herausgezogenen Menge des Bandes verbunden ist, und in welchem eine Einrichtung (46) vorgesehen Ist, die das Band gegen die Welle in einen Reibungskontakt drückt,
**dadurch gekennzeichnet,**
**dass** die Einrichtung zum Drücken einen Kragen (46) aufweist, der um die Welle (44) herum angeordnet ist, und
**dass** das Band zwischen dem Kragen und der Welle hindurch läuft.

2. Gerät nach Anspruch 1,
In welchem das Band (11) ein feststehendes Endelement (14) und ein relativ zu diesem bewegliches Element (16) besitzt, welches durch einen Benutzer längs des Bandes beweglich ist, aber In einer relativ zum Band elnjustierten Position verbleibt.

3. Gerät nach Anspruch 2,
in welchem das relativ bewegliche Element (16) eine Hakeneinrichtung (21) einschließt, wobei das Endelement (14) so angeordnet ist, dass es In die Hakeneinrichtung hineinpasst.

4. Gerät nach einem der vorstehenden Ansprüche,
In welchem das Gerät ein Gehäuse (12, 22) aufweist, dessen Äußeres einen Weg (32, 82) bildet, längs welchem das Band platziert werden kann, um eine bekannte Größe für Kalibrierzwecke zu definieren.

5. Gerät nach Anspruch 2 und 4,
In welchem das Ende des Weges durch einen Einschnitt (33) definiert wird, der so dimensioniert ist, dass er entweder das Endelement (14) oder eine Kombination des Endelementes (14) und des relativ beweglichen Elementes (16) in Kontakt miteinander einpasst.

6. Gerät nach Anspruch 1,
in welchem das Gerät ein Gehäuse (72) aufweist, und in welchem ein Element (76) an dem Gehäuse zum Bilden einer Messposition angebracht ist.

7. Gerät nach Anspruch 6,
in welchem das Element (76) schwenkbar an dem Gehäuse (72) angebracht ist.

8. Gerät nach Anspruch 6 oder 7,
In welchem das Äußere des Gehäuses (72) einen Weg (82) bildet, längs welchem das Band (71) platziert werden kann, um eine bekannte Größe für Kalibrierzwecke zu definieren.

9. Gerät nach Anspruch 7 und 8,
In welchem das Element (76) In eine erste Position zum Bilden der Messposition schwenkbar und in eine zweite Position zum Bilden des Endes des äußeren Kalibrierweges (82) schwenkbar ist.

10. Gerät nach Anspruch 6,
In welchem das Element fest an dem Gehäuse angebracht ist.

11. Gerät nach Anspruch 10,
In weichem das Element eine Hakeneinrichtung einschließt.

12. Gerät nach Anspruch 10,
In welchem das Element einen oder mehrere Einschnitte aufweist, die an oder benachbart zu einem Abschnitt (28) des Gehäuses angebracht sind, aus dem das Band (111) austritt.

13. Gerät nach einem der Ansprüche 6 bis 12,
in welchem das Äußere des Gehäuses eine Formation (33, 76, 100) besitzt, relativ zu welcher, nachdem eine Länge des Bandes herausgezogen und um den Umfang (32, 82) des Gehäuses gewunden ist, das Element in einer wiederholbaren Welse akkurat positioniert werden kann.

14. Verfahren zum Messen eines Abstandes unter Verwendung eines Gerätes (10, 70) nach einem der vorstehenden Ansprüche.

## Revendications

1. Dispositif de mètre ruban électronique (10, 70, 90) comprenant un ruban (11, 71,111) qui passe autour d'un axe rotatif (44), l'axe étant connecté à un dispositif codeur (45) permettant de mesurer la quantité de ruban tiré, et dans lequel des moyens (46) sont fournis pour contraindre le ruban à s'engager dans l'axe par frottement, **caractérisé en ce que** les moyens de contrainte comprennent une bague d'arrêt (46) agencée autour de l'axe (44), et **en ce que** le ruban passe entre la bague d'arrêt et l'axe.

2. Dispositif selon la revendication 1, dans lequel le ruban (11) a un élément d'extrémité fixe (14) et un élément relativement mobile (16), qui peut être déplacé par un utilisateur le long du ruban mais qui reste dans une position ajustée par rapport au ruban.

3. Dispositif selon la revendication 2, dans lequel l'élément relativement mobile (16) inclut un moyen formant crochet (21), l'élément d'extrémité (14) étant agencé pour s'ajuster à l'intérieur du moyen formant crochet.

4. Dispositif selon l'une quelconque des revendications précédentes, dans lequel le dispositif comprend un boîtier (12, 22), dont l'extérieur définit un trajet (32, 82) le long duquel le ruban peut être placé pour définir une dimension connue à des fins d'étalonnage.

5. Dispositif selon les revendications 2 et 4, dans lequel l'extrémité du trajet est définie par un logement (33), qui est dimensionné pour s'ajuster soit à l'élément d'extrémité (14) soit à une combinaison de l'élément d'extrémité (14) et de l'élément relativement mobile (16) en contact l'un avec l'autre.

6. Dispositif selon la revendication 1, dans lequel le dispositif comprend un boîtier (72) et un élément (76) est attaché au boîtier pour définir une position de mesure.

7. Dispositif selon la revendication 6, dans lequel l'élément (76) est attaché au boîtier (72) de manière pivotante.

8. Dispositif selon la revendication 6 ou 7, dans lequel l'extérieur du boîtier (72) définit un trajet (82) le long duquel le ruban (71) peut être placé pour définir une dimension connue à des fins d'étalonnage.

9. Dispositif selon les revendications 7 et 8, dans lequel ledit élément (76) peut pivoter vers une première position pour définir ladite position de mesure, et peut pivoter vers une deuxième position pour définir l'extrémité dudit trajet d'étalonnage extérieur (82).

10. Dispositif selon la revendication 6, dans lequel ledit élément est attaché au boîtier de manière fixe.

11. Dispositif selon la revendication 10, dans lequel ledit élément inclut un moyen formant crochet.

12. Dispositif selon la revendication 10, dans lequel l'élément comprend un ou plusieurs logements agencés au niveau de, ou de façon adjacente à la partie (28) du boîtier à partir de laquelle le ruban (111) émerge.

13. Dispositif selon l'une quelconque des revendications 6 à 12, dans lequel l'extérieur du boîtier a une forme (33,76, 100) par rapport à laquelle, après qu'une longueur de ruban a été tirée et enroulée autour de la périphérie (32, 82) du boîtier, l'élément peut être localisé avec précision de façon connue.

14. Procédé de mesure d'une distance utilisant un dispositif (10, 70) selon l'une quelconque des revendications précédentes.
